(19) Europäisches Patentamt
European Patent Office
Office européen des brevets

(11) Veröffentlichungsnummer : **0 171 689**
**B1**

(12) # EUROPÄISCHE PATENTSCHRIFT

(45) Veröffentlichungstag der Patentschrift : **11.11.87**

(21) Anmeldenummer : **85109431.8**

(22) Anmeldetag : **26.07.85**

(51) Int. Cl.[4] : **C 07 C 91/16**, C 07 C 93/14, C 07 C 91/34, C 07 D231/38, C 07 D231/56, C 07 C 97/10

(54) **Neue Aminoalkohole, Verfahren zu deren Herstellung sowie diese Verbindungen enthaltende Arzneimittel und Zwischenprodukte.**

(30) Priorität : **02.08.84 DE 3428526**

(43) Veröffentlichungstag der Anmeldung : **19.02.86 Patentblatt 86/08**

(45) Bekanntmachung des Hinweises auf die Patenterteilung : **11.11.87 Patentblatt 87/46**

(84) Benannte Vertragsstaaten : **AT BE CH DE FR GB IT LI LU NL SE**

(56) Entgegenhaltungen :
**EP-A- 0 002 867**
**EP-A- 0 042 593**
**DE-A- 2 704 895**
**LIEBIGS ANNALEN DER CHEMIE, Band 742, 1970, Verlag Chemie GmbH, Weinheim, DE; K.H. MAGOSCH et al.: "Anlagerung von Epoxiden an cyclische Amidine"**
**Die Akte enthält technische Angaben, die nach dem Eingang der Anmeldung eingereicht wurden und die nicht in dieser Patentschrift enthalten sind.**

(73) Patentinhaber : **Boehringer Mannheim GmbH**
**Sandhofer Strasse 116**
**D-6800 Mannheim 31 (DE)**

(72) Erfinder : **Friebe, Walter-Gunar, Dr.**
**Heidelberger Landstrasse 111 d**
**D-6100 Darmstadt 13 (DE)**
Erfinder : **Kampe, Wolfgang, Dr. rer. nat.**
**Zedernstrasse 49**
**D-6805 Heddesheim (DE)**
Erfinder : **Böhm, Erwin, Dr. med.**
**In der Schanz 37**
**D-6905 Schriesheim (DE)**
Erfinder : **Strein, Klaus, Prof.**
**Eichenstrasse 45**
**D-6944 Hemsbach (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Aminoalkohole, Verfahren zu deren Herstellung sowie diese Verbindungen enthaltende Arzneimittel.

Die erfindungsgemäßen Verbindungen besitzen eine signifikante Wirkung auf die Verformbarkeit der Erythrozyten und verbessern die Fließeigenschaften des Blutes. Sie können daher insbesondere zur Behandlung von peripheren, cerebralen und coronaren Durchblutungsstörungen eingesetzt werden.

Desweiteren zeigen sie eine positiv inotrope Wirkung.

Ähnliche Verbindungen sind in EP-A-0 002 867 und EP-A-0 042 593 beschrieben. Jedoch ist dort kein Hinweis auf eine rheologische Wirkung enthalten.

Die vorliegende Erfindung betrifft Aminoalkohole der allgemeinen Formel I

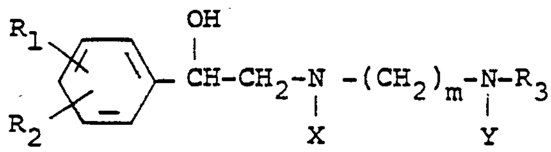

(I)

in der

$R_1$ und $R_2$ gleich oder verschieden sind und jeweils Wasserstoff, Hydroxy-, $C_1$- bis $C_4$-Alkoxy-, Benzyloxy-, $C_1$- bis $C_4$-Alkoxycarbonyl-, $C_1$- bis $C_4$-Alkansulfonamido-, $C_1$- bis $C_4$-Alkansulfinyl- oder $C_1$- bis $C_4$-Alkansulfonylgruppen,

m die Zahl 2, 3 oder 4,

X und Y gleich oder verschieden sind und jeweils Wasserstoff oder eine Benzylgruppe und

$R_3$ einen zweifach durch $C_1$- bis $C_6$-Alkyl substituierten Phenylrest, einen Nitrophenylrest, einen Aminophenylrest, einen 1,3,5-Tri-$C_1$- bis $C_6$-alkyl-2,4-dioxo-1H, 3H-pyrimidin-6-ylrest, einen Indolylrest, einen Indazolylrest, einen Benzimidazolylrest, einen 1,4-Di-$C_1$- bis $C_6$-alkyl-pyrazol-5-ylrest oder einen Rest Q

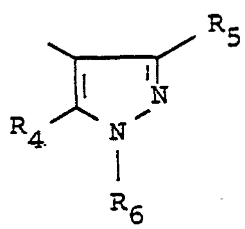

(Q)

worin $R_4$ und $R_5$ gleich oder verschieden sind und jeweils fuer $C_1$- bis $C_4$-Alkylreste stehen und $R_6$ einen Rest aus der folgenden Gruppe : $C_1$- bis $C_6$-Alkyl, $C_1$- bis $C_6$-Alkenyl, Phenyl und Benzyl darstellt, bedeuten, deren pharmakologisch vertraegliche Salze, Verfahren zu ihrer Herstellung und diese Verbindungen enthaltende Arzneimittel.

Weiterhin betrifft die Erfindung pharmazeutische Praeparate mit einem Gehalt an Verbindungen der allgemeinen Formel I sowie die Verwendung von Verbindungen der allgemeinen Formel I zur Herstellung solcher Praeparate.

Außer den in den Beispielen genannten Verbindungen sind Gegenstand der Erfindung insbesondere alle Substanzen, die jede moegliche Kombination der in den Beispielen genannten Substituenten aufweisen.

Das erfindungsgemaeße Verfahren zur Herstellung der Verbindungen der Formel I ist dadurch gekennzeichnet, daß man in an sich bekannter Weise eine Verbindung der allgemeinen Formel II

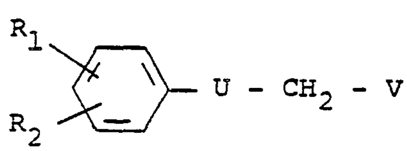

(II)

in der

$R_1$ und $R_2$ die obengenannte Bedeutung haben, waehrend

V einen reaktiven Rest darstellt und

U die Gruppe $>$C=O oder $>$CH—Z bedeutet, wobei

Z eine Hydroxygruppe oder auch gemeinsam mit V ein Sauerstoffatom sein kann,

mit einer Verbindung der allgemeinen Formel III

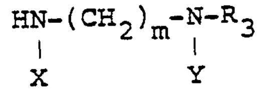

(III)

in der X, Y, m und $R_3$ die obengenannte Bedeutung haben, umsetzt und fuer den Fall, daß U die Gruppe $>C=O$ bedeutet, anschließend reduziert, eine fuer X und/oder Y stehende Benzylgruppe gewuenschtenfalls abspaltet, einen fuer $R_1$ oder $R_2$ stehenden Rest gewuenschtenfalls in einen anderen, dem Anspruch entsprechenden Rest umwandelt, und gegebenenfalls die so erhaltenen Verbindungen der allgemeinen Formel I in ihre pharmakologisch vertraeglichen Salze ueberfuehrt.

Die als Zwischenprodukte anfallenden Verbindungen der Formel Ia

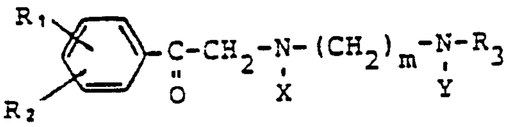

(Ia)

in der $R_1$, $R_2$, m, X und Y sowie $R_3$ die oben angegebene Bedeutung haben, sind neu und ebenfalls Gegenstand der Erfindung.

Als reaktive Reste V der Verbindungen der allgemeinen Formel II kommen Chlor, Brom, Mesyloxy und Tosyloxy in Frage.

Das erfindungsgemaeße Verfahren wird zweckmaeßig in einem unter den Reaktionsbedingungen inerten organischen Loesungsmittel, z. B. Toluol, Dioxan, Tetrahydrofuran, Ethylenglykoldimethylether, Ethanol, Butanol, Aceton oder Dimethylformamid, gegebenenfalls in Gegenwart eines saeurebindenden Mittels, durchgefuehrt. Auch Mischungen der vorgenannten Loesungsmittel sind geeignet.

Die gegebenenfalls vorzunehmende Reduktion einer fuer U stehenden $>C=O$-Gruppe erfolgt mittels komplexer Metallhydride wie beispielsweise Natriumborhydrid oder durch katalytische Hydrierung an Edelmetallkatalysatoren.

Eine allfaellige Abspaltung einer fuer X und Y stehenden Benzylgruppe wird zweckmaeßig mit katalytisch erregtem Wasserstoff durchgefuehrt.

Als nachtraegliche Umwandlung eines Restes $R_1$ oder $R_2$ in einem anderen, durch den Anspruch definierten Rest $R_1$ oder $R_2$ seien beispielsweise genannt:

eine hydrogenolytische Umwandlung einer Benzyloxygruppe in eine Hydroxygruppe.

Die Verbindungen der Formeln II und III sind literaturbekannt oder koennen nach trivialen Methoden aus literaturbekannten Stoffen hergestellt werden, vgl. DE-OS 3 023 369 und 3 131 146.

Zur Ueberfuehrung der Verbindungen der Formel I in ihre pharmakologisch vertraeglichen Salze setzt man sie vorzugsweise in einem organischen Loesungsmittel mit einer anorganischen oder organischen Saeure, z. B. Salzsaeure, Bromwasserstoffsaeure, Phosphorsaeure, Schwefelsaeure, Essigsaeure, Zitronensaeure, Weinsaeure, Maleinsaeure, Fumarsaeure, Benzoesaeure oder Oxalsaeure um.

Die erfindungsgemaeßen Verbindungen der Formel I koennen in Form eines racemischen Gemisches anfallen. Die Trennung des Racemats in die optisch aktiven Formen geschieht nach an sich bekannten Methoden ueber die diastereomeren Salze aktiver Saeuren, wie z. B. Weinsaeure, Aepfelsaeure oder Camphersulfonsaeure.

Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielweise als Tabletten oder Dragees ausgeformt oder unter Zugabe entsprechender Hilfsstoffe in Wasser oder Oel, wie z. B. Olivenoel, suspendiert oder geloest.

Die erfindungsgemaeßen neuen Substanzen der allgemeinen Formel I und ihre Salze koennen in fluessiger Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z. B. Tartrat- und Citratpuffer, Ethanol, Komplexbildner (wie Ethylendiamintetraessigsaeure und deren nichttoxische Salze), hochmolekulare Polymere (wie fluessiges Polyethylenoxid) zur Viskositaetsregulierung. Feste Traegerstoffe sind z. B. Staerke, Lactose, Mannit, Methylcellulose, Talkum, hochdisperse Kieselsaeuren, hochmolekulare Fettsaeuren (wie Sterinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlung und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendungen pro Tag wirksam, um die gewünschten Resultate zu erhalten.

Bevorzugt im Sinne der Anmeldung sind außer den in den Beispielen genannten Verbindungen die folgenden:

2-Hydroxy-5-<1-hydroxy-2-[2-(2,6-dimethyl-phenylamino) ethyl-amino] ethyl>benzolsulfonamid
N-[[2-Hydroxy-5-<1-hydroxy-2-[2-(2,6-dimethyl-phenylamino)-ethylamino] ethyl>phenyl]] methansulfonamid
4-Hydroxy-3-methylsulfinyl-α-<[2-(2,6-dimethyl-phenylamino)-ethyl] aminomethyl>phenylmethanol
4-Hydroxy-3-methylsulfonyl-α<[2-(2,6-dimethyl-phenylamino)-ethyl] aminomethyl>phenylmethanol
3-Hydroxy-α-<[2-(3-nitro-phenylamino) ethyl] aminomethyl>phenylmethanol
4-Hydroxy-α-<[2-(3-nitro-phenylamino) ethyl] aminomethyl>phenylmethanol
3-Hydroxy-α-<[2-(2,4-dioxo-1,3,5-trimethyl-1H,3H-pyrimidin-6-ylamino) ethyl] aminomethyl>phenylmethanol
α-<[2-(1-Allyl-3,5-dimethyl-pyrazol-4-ylamino) ethyl] aminomethyl>phenylmethanol
3-Hydroxy-α-<[2-(1-allyl-3,5-dimethyl-pyrazol-4-ylamino) ethyl]-aminomethyl>phenylmethanol
4-Hydroxy-α-<[2-(1-allyl-3,5-dimethyl-pyrazol-4-ylamino) ethyl]-aminomethyl>phenylmethanol
α-<[2-(1-Phenyl-3,5-dimethyl-pyrazol-4-ylamino) ethyl] aminomethyl>phenylmethanol
α-<[2-(1-Benzyl-3,5-dimethyl-pyrazol-4-ylamino) ethyl] aminomethyl>phenylmethanol

## Beispiel 1

α-<[2-(3-Nitro-phenylamino) ethyl] aminomethyl>phenylmethanol

Eine Mischung aus 3.0 g (25 mmol) Styroloxid, 9.0 g (50 mmol) N-(3-Nitro-phenyl)-1,2-diaminoethan und 25 ml n-Butanol wird 3 d bei Raumtemperatur geruehrt. Man versetzt mit 100 ml Diethylether, filtriert den Niederschlag ab und kristallisiert aus Ethylacetat um. Es verbleiben 3.9 g der Titelverbindung (52 % d. Th.) vom Schmp. 141-142 °C.

## Beispiel 2

In analoger Weise wie in Beispiel 1 beschrieben erhaelt man :

| | Bezeichnung | Ausbeute % | Schmelzpunkt °C (Lösungsmittel) |
|---|---|---|---|
| a) | 2-Benzyloxy-α-<[2-(2,6-dimethyl-phenylamino)ethyl]aminomethyl>-phenylmethanol aus 2-Benzyloxy-styroloxid und N-(2,6-Dimethyl-phenyl)-1,2-diamino-ethan | 59 | Öl |
| b) | 2-Methoxy-α-<[2-(2,6-dimethyl-phenylamino)ethyl]-N-benzyl-aminomethyl>phenylmethanol aus 2-Methoxy-styroloxid und N-[2-(2,6-Dimethyl-phenylamino)ethyl]-benzylamin | 29 | Öl |

## Beispiel 3

4-Benzyloxy-α-<[2-(2,6-dimethyl-phenylamino) ethyl] aminomethyl>-phenylmethanol

Zu einer Loesung von 33.8 g (80 mmol) 1-(4-Benzyloxy-phenyl)-2-[2-(2,6-dimethyl-phenylamino) ethylamino] ethanon in 600 ml Methanol gibt man bei 0 °C innerhalb 10 min 9.6 g (0.24 mol) Natriumborhydrid, ruehrt 1 h bei 0 °C nach, gießt auf Eis, stellt mit verd. Essigsaeure sauer, versetzt mit ueberschuessiger Natriumhydrogencarbonatloesung und extrahiert mit Dichlormethan. Nach Einengen des Extrakts verbleiben 28.9 g der Titelverbindung (87 % d. Th.) als Oel.

## Beispiel 4

In analoger Weise wie in Beispiel 3 beschrieben erhaelt man als Oele :

| | Bezeichnung | Ausbeute % |
|---|---|---|
| a) | 3-Benzyloxy-α-<[2-(2,6-dimethyl-phenylamino)ethyl]aminomethyl>-phenylmethanol aus 1-(3-Benzyloxy-phenyl)-2-[2-(2,6-dimethyl-phenylamino)ethylamino]-ethanon | 45 |
| b) | α-<[2-(2,6-Dimethyl-phenylamino)-ethyl]-N-benzyl-aminomethyl>-phenylmethanol aus 2-[2-(2,6-Dimethyl-phenylamino)-ethyl-N-benzylamino]-1-phenyl-ethanon | 84 |
| c) | 4-Benzyloxy-3-methoxy-α-<[2-(2,6-dimethyl-phenylamino)ethyl]-N-benzyl-aminomethyl>phenylmethanol aus 1-(4-Benzyloxy-3-methoxy-phenyl)-2-[2-(2,6-dimethyl-phenylamino)ethyl-N-benzylamino]ethanon | 71 |
| d) | 3,5-Dibenzyloxy-α-<[2-(2,6-dimethyl-phenylamino)ethyl]-N-benzyl-amino-methyl>phenylmethanol aus 1-(3,5-Dibenzyloxy-phenyl)-2-[2-(2,6-dimethyl-phenylamino)ethyl-N-benzyl-amino]ethanon | 55 |
| e) | α-<[3-(2,6-Dimethyl-phenylamino)propyl]-N-benzyl-aminomethyl>phenylmethanol aus 2-[3-(2,6-Dimethyl-phenylamino)propyl-N-benzylamino]-1-phenyl-ethanon | 76 |
| f) | 4-Benzyloxy-α-<[3-(2,6-dimethyl-phenylamino)propyl]-N-benzyl-amino-methyl>phenylmethanol aus 1-(4-Benzyloxy-phenyl)-2-[3-(2,6-dimethyl-phenylamino)propyl-N-benzylamino]ethanon | 68 |
| g) | 3-Benzyloxy-α-[[<2-[(1,3,5-trimethyl-pyrazol-4-yl)-N-benzylamino]ethyl>-N-benzyl-aminomethyl]]phenylmethanol aus 1-(3-Benzyloxy-phenyl)-2-<2-[1,3,5-trimethyl-pyrazol-4-yl)-N-benzylamino]-ethyl-N-benzylamino>ethanon | 62 |
| h) | 4-Benzyloxy-α-[[<2-[(1,3,5-trimethyl-pyrazol-4-yl)-N-benzylamino]ethyl>-N-benzyl-aminomethyl]]phenylmethanol aus 1-(4-Benzyloxy-phenyl)-2-<2-[(1,3,5-trimethyl-pyrazol-4-yl)-N-benzylamino]-ethyl-N-benzylamino>ethanon | 55 |

(Fortsetzung)

| | Bezeichnung | Ausbeute % |
|---|---|---|
| i) | α-[[<2-[(1,3,5-Trimethyl-pyrazol-4-yl)-N-benzylamino]ethyl>-N-benzyl-aminomethyl]]-phenylmethanol aus<br>1-Phenyl-2-<2-[(1,3,5-trimethyl-pyrazol-4-yl)-N-benzylamino]ethyl-N-benzylamino>-ethanon | 45 |
| j) | 3-Benzyloxy-α-<[2-(1,4-dimethyl-pyrazol-5-ylamino)ethyl]-N-benzyl-aminomethyl>-phenylmethanol aus<br>1-(3-Benzyloxy-phenyl)-2-[2-(1,4-dimethyl-pyrazol-5-ylamino)ethyl-N-benzylamino]-ethanon | 38 |
| k) | 4-Benzyloxy-α-<[2-(1,4-dimethyl-pyrazol-5-ylamino)ethyl]-N-benzyl-aminomethyl>-phenylmethanol aus<br>1-(4-Benzyloxy-phenyl)-2-[2-(1,4-dimethyl-pyrazol-5-ylamino)ethyl-N-benzylamino]-ethanon | 43 |
| l) | 3-Benzyloxy-α-<[2-(indazol-4-ylamino)-ethyl]aminomethyl>phenylmethanol aus<br>1-(3-Benzyloxy-phenyl)-2-[2-(indazol-4-ylamino)ethylamino]ethanon | 35 |
| m) | 4-Benzyloxy-α-<[2-(indazol-4-ylamino)-ethyl]-N-benzyl-aminomethyl>phenyl-methanol aus<br>1-(4-Benzyloxy-phenyl)-2-[2-(indazol-4-ylamino)ethyl-N-benzylamino]ethanon | 88 |

## Beispiel 5

α-<[2-(2,6-Dimethyl-phenylamino) ethyl] aminoethyl>-2-hydroxyphenylmethanol

Eine Loesung von 6.8 g (18 mmol) 2-Benzyloxy-α-<[2-(2,6-dimethyl-phenylamino) ethyl] aminomethyl> phenylmethanol (Verbindung des Beispiels 2 a) in 100 ml Ethanol und 10 ml Wasser wird bei Raumtemperatur und 1 bar Wasserstoffdruck ueber 1 g 10 proz. Palladiumkohle hydriert. Man filtriert, engt ein, nimmt in Aceton auf und versetzt mit der berechneten Menge Oxalsaeure. Nach Umkristallisation aus Aceton erhaelt man 5.4 g des Oxalats der Titelverbindung (77 % d. Th.) vom Schmp. 112-113 °C.

## Beispiel 6

In analoger Weise wie in Beispiel 5 beschrieben erhaelt man :

(Siehe Tabelle Seite 7 ff.)

| | Bezeichnung | Ausbeute % | Schmelzpunkt °C (Lösungsmittel) |
|---|---|---|---|
| a) | α-<[2-(2,6-Dimethyl-phenylamino)ethyl]-aminomethyl>-2-methoxy-phenylmethanol aus Verbindung des Beispiels 2 b | 83 | Oxalat 186-188 (2-Propanol) |
| b) | α-<[2-(2,6-Dimethyl-phenylamino) ethyl]aminomethyl>-4-hydroxy-phenylmethanol aus Verbindung des Beispiels 3 | 54 | 159-160 (Diethylether) |
| c) | α-<[2-(2,6-Dimethyl-phenylamino)-ethyl]aminomethyl>-3-hydroxy-phenylmethanol aus Verbindung des Beispiels 4 a | 65 | Oxalat 85-90 (amorph) (Aceton) |
| d) | α-<[2-(2,6-Dimethyl-phenylamino)-ethyl]aminomethyl>phenylmethanol aus Verbindung des Beispiels 4 b | 43 | 88-90 (2-Propanol) |
| e) | α-<[2-(2,6-Dimethyl-phenylamino)-ethyl]aminomethyl>-4-hydroxy-3-methoxy-phenylmethanol aus Verbindung des Beispiels 4 c | 56 | Hydrochlorid 168-170 (Ethanol) |
| f) | 3,5-Dihydroxy-α-<[2-(2,6-dimethyl-phenylamino)ethyl]aminomethyl>-phenylmethanol aus Verbindung des Beispiels 4 d | 75 | Hydrochlorid 100-110 (amorph) (Ethylacetat) |
| g) | α-<[3-(2,6-Dimethyl-phenylamino)-propyl]aminomethyl>phenylmethanol aus Verbindung des Beispiels 4 e | 85 | 93-94 (2-Propanol) |
| h) | α-<[3-(2,6-Dimethyl-phenylamino)-propyl]aminomethyl>-4-hydroxy-phenylmethanol aus Verbindung des Beispiels 4 f | 76 | 148-150 (2-Propanol) |
| i) | 3-Hydroxy-α-<[2-(1,3,5-trimethyl-pyrazol-4-ylamino)ethyl]amino-methyl>phenylmethanol aus Verbindung des Beispiels 4 g | 48 | Hydrochlorid 65-70 (amorph) (2-Propnaol) |
| j) | 4-Hydroxy-α-<[2-(1,3,5-trimethyl-pyrazol-4-ylamino)ethyl]amino-methyl>phenylmethanol aus Verbindung des Beispiels 4 h | 45 | 162-164 (Diethylether) |

(Fortsetzung)

| | Bezeichnung | Ausbeute % | Schmelzpunkt °C (Lösungsmittel) |
|---|---|---|---|
| k) | α-<[2-(1,3,5-Trimethyl-pyrazol-4-ylamino)ethyl]aminomethyl>phenyl-methanol aus Verbindung des Beispiels 4 i | 51 | Dihydrochlorid 75-80 (amorph) |
| l) | α-<[2-(1,4-Dimethyl-pyrazol-5-ylamino)ethyl]aminomethyl>-3-hydroxy-phenylmethanol aus Verbindung des Beispiels 4 j | 60 | Dimaleat 146-149 (Aceton) |
| m) | α-<[2-(1,4-Dimethyl-pyrazol-5-ylamino)ethyl]aminomethyl>-4-hydroxy-phenylmethanol aus Verbindung des Beispiels 4 k | 77 | Dimaleat 142-144 (Aceton) |
| n) | 3-Hydroxy-α-<[2-(indazol-4-yl-amino)ethyl]aminomethyl>phenyl-methanol aus Verbindung des Beispiels 4 l | 93 | 90-95 (amorph) (Diethylether) |
| o) | 4-Hydroxy-α-<[2-(indazol-4-yl-amino)ethyl]aminomethyl>phenyl-methanol aus Verbindung des Beispiels 4 m | 82 | 89-90 (amorph) (Diethylether) |

Beispiel 7

Das in Beispiel 3 verwendete Zwischenprodukt 1-(4-Benzyloxy-phenyl)-2-[2-(2,6-dimethyl-phenylamino) ethylamino]-ethanon kann wie folgt hergestellt werden :

Eine Mischung aus 6.6 g (40 mmol) 2-(2,6-Dimethyl-phenylamino) ethylamin, 12.2 g (40 mmol) 4-Benzyloxy-phenacylbromid, 150 ml Tetrahydrofuran und 5.1 g Triethylamin wird 3 h bei Raumtemperatur geruehrt. Danach wird filtriert und im Vakuum eingeengt. Es verbleiben 15.3 g der Titelverbindung (99 % d. Th.) als Oel.

Beispiel 8

In analoger Weise wie in Beispiel 7 beschrieben erhaelt man als Oele :

(Siehe Tabelle Seite 9 ff.)

| | Bezeichung | Ausbeute % |
|---|---|---|
| a) | 1-(3-Benzyloxy-phenyl)-2-[2-(2,6-dimethyl-phenylamino)ethylamino]ethanon aus 2-(2,6-Dimethyl-phenylamino)ethylamin und 3 Benzyloxy-phenacylbromid | 96 |
| b) | 2-[2-(2,6-Dimethyl-phenylamino)ethyl-N-benzylamino]-1-phenyl-ethanon aus N-[2-(2,6-Dimethyl-phenylamino)ethyl]-benzylamin und Phenacylbromid | 97 |
| c) | 1-(4-Benzyloxy-3-methoxy-phenyl)-2-[2-(2,6-dimethyl-phenylamino)ethyl-N-benzylamino]ethanon aus N-[2-(2,6-Dimethyl-phenylamino)-ethyl]benzylamin und 4-Benzyloxy-3-methoxy-phenacyl-bromid | 98 |
| d) | 1-(3,5-Dibenzyloxy-phenyl)-2-[2-(2,6-dimethyl-phenylamino)ethyl-N-benzylamino]-ethanon aus N-[2-(2,6-Dimethyl-phenylamino)ethyl]-benzylamin und 3,5-Dibenzyloxy-phenacyl-bromid | 95 |
| e) | 2-[3-(2,6-Dimethyl-phenylamino)propyl-N-benzylamino]-1-phenyl-ethanon aus N-[3-(2,6-Dimethyl-phenylamino)-propyl]benzylamin und Phenacylbromid | 100 |
| f) | 1-(4-Benzyloxy-phenyl)-2-[3-(2,6-dimethyl-phenylamino)propyl-N-benzylamino]ethanon aus N-[3-(2,6-Dimethyl-phenylamino)propyl]-benzylamin und 4-Benzyloxy-phenacylbromid | 95 |
| g) | 1-(3-Benzyloxy-phenyl)-2-<2-[(1,3,5-trimethyl-pyrazol-4-yl)-N-benzylamino]ethyl-N-benzyl-amino>ethanon aus N-<2-[(1,3,5-Triemthyl-pyrazol-4-yl)-N-benzylamino]ethyl>benzylamin und 3-Benzyl-oxy-phenacylbromid | 95 |
| h) | 1-(4-Benzyloxy-phenyl)-2-<2-[(1,3,5-trimethyl-pyrazol-4-yl)-N-benzylamino]ethyl-N-benzyl-amino>ethanon aus N-<2-[(1,3,5-Trimethyl-pyrazol-4-yl)-N-benzylamino]ethyl>benzylamin und 4-Benzyl-oxy-phenacylbromid | 95 |

(Fortsetzung)

| | Bezeichnung | Ausbeute % |
|---|---|---|
| i) | 1-Phenyl-2-<2-[(1,3,5-trimethyl-pyrazol-4-yl)-N-benzylamino]ethyl-N-benzylamino>-ethanon aus N-<2-[(1,3,5-Trimethyl-pyrazol-4-yl)-N-benzylamino]ethyl>benzylamin und Phenacylbromid | 98 |
| j) | 1-(3-Benzyloxy-phenyl)-2-[2-(1,4-dimethyl-pyrazol-5-ylamino)ethyl-N-benzylamino]-ethanon aus N-[2-(1,4-Dimethyl-pyrazol-5-ylamino)-ethyl]benzylamin und 3-Benzyloxy-phenacylbromid | 100 |
| k) | 1-(4-Benzyloxy-phenyl)-2-[2-(1,4-dimethyl-pyrazol-5-ylamino)ethyl-N-benzylamino]ethanon aus N-[2-(1,4-Dimethyl-pyrazol-5-ylamino)ethyl]benzylamin und 4-Benzyloxy-phenacylbromid | 92 |
| l) | 1-(3-Benzyloxy-phenyl)-2-[2-(indazol-4-ylamino)ethylamino]ethanon aus 2-(Indazol-4-ylamino)ethylamin und 3-Benzyloxy-phenacylbromid | 94 |
| m) | 1-(4-Benzyloxy-phenyl)-2-[2-(indazol-4-ylamino)ethyl-N-benzyl-amino]ethanon aus N-[2-(Indazol-4-ylamino)ethyl]benzylamin und 4-Benzyloxy-phenacylbromid | 85 |

## Beispiel 9

Es wurden Tabletten hergestellt: Jede Tablette enthält 10 mg α-<[2-(3-Nitro-phenylamino) ethyl] aminomethyl>-phenylmethanol. Die Tabletten wurden gemäß der folgenden Rezeptur hergestellt:

| | |
|---|---|
| α-<[2-(3-Nitro-phenylamino) ethyl]-aminomethyl> phenylmethanol | 10 g |
| Lactose | 80 g |
| Stärke | 29 g |
| Magnesiumstearat | 1 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1 000 Tabletten mit einem Einzelgewicht von 0,12 g verpreßt.

**Patentansprüche**

1. Aminoalkohole der allgemeinen Formel I

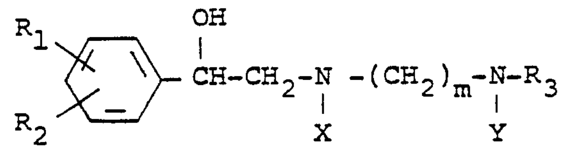

(I)

in der

$R_1$ und $R_2$ gleich oder verschieden sind und jeweils Wasserstoff, Hydroxy-, $C_1$- bis $C_4$-Alkoxy-, Benzyloxy-, $C_1$- bis $C_4$-Alkoxycarbonyl-, $C_1$- bis $C_4$-Alkansulfonamido-, $C_1$- bis $C_4$-Alkansulfinyl- oder $C_1$- bis $C_4$-Alkansulfonylgruppen,

m die Zahl 2, 3 oder 4,

X und Y gleich oder verschieden sind und jeweils Wasserstoff oder eine Benzylgruppe und

$R_3$ einen zweifach durch $C_1$- bis $C_6$-Alkyl substituierten Phenylrest, einen Nitrophenylrest, einen Aminophenylrest, einen 1,3,5-Tri-$C_1$- bis $C_6$-alkyl-2,4-dioxo-1H, 3H-pyrimidin-6-ylrest, einen Indolylrest, einen Indazolylrest, einen Benzimidazolylrest, einen 1,4-Di-$C_1$- bis $C_6$-alkyl-pyrazol-5-ylrest oder einen Rest Q

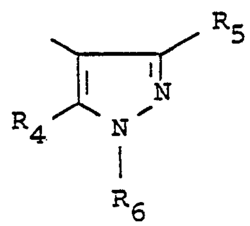

(Q)

worin $R_4$ und $R_5$ gleich oder verschieden sind und jeweils fuer $C_1$- bis $C_4$-Alkylreste stehen und $R_6$ einen Rest aus der folgenden Gruppe : $C_1$- bis $C_6$-Alkyl, $C_1$- bis $C_6$-Alkenyl, Phenyl und Benzyl darstellt, bedeuten, sowie deren pharmakologisch verträgliche Salze.

2. Verbindungen gemäß Anspruch 1, in denen m, X, Y und $R_3$ die angegebene Bedeutung haben und $R_1$ und $R_2$ gleich oder verschieden sind und jeweils Wasserstoff, Hydroxy, Methoxy oder Benzyloxy darstellen.

3. Verbindungen gemäß Anspruch 1 oder 2, in denen $R_1$, $R_2$, m, X und Y die angegebene Bedeutung, haben und $R_3$ einen 2,6-Dimethyl-phenyl-, 3-Nitrophenyl-, Indazolyl-, 1,4-Dimethyl-pyrazol-5-yl-, 2,4-Dioxo-1,3,5-trimethyl-1H,3H-pyrimidin-6-yl-Rest oder einen Rest Q

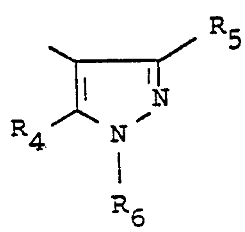

(Q)

worin $R_4$ und $R_5$ jeweils Methyl und $R_6$ Methyl, Allyl, Phenyl oder Benzyl bedeuten, darstellen.

4. Verbindungen gemäß Anspruch 1, in denen

$R_1$ Wasserstoff, Hydroxy, Methoxy oder Benzyloxy,

$R_2$ Wasserstoff, Hydroxy, Methoxy oder Benzyloxy,

m die Zahlen 2 oder 3,

X Wasserstoff oder Benzyl,

Y Wasserstoff oder Benzyl und

$R_3$ einen 2,6-Dimethyl-phenyl-, 3-Nitrophenyl-, Indazolyl-, 1,4-Dimethyl-pyrazol-5-yl oder 1,3,5-Trimethyl-pyrazol-4-yl-Rest

darstellen.

5. Verfahren zur Herstellung von Aminoalkoholen der allgemeinen Formel I

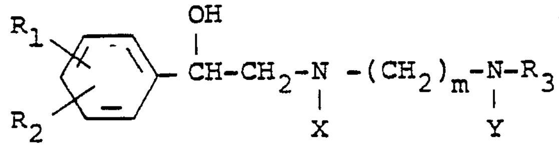

(I)

in der

$R_1$ und $R_2$ gleich oder verschieden sind und jeweils Wasserstoff, Hydroxy-, $C_1$- bis $C_4$-Alkoxy-, Benzyloxy-, $C_1$- bis $C_4$-Alkoxycarbonyl-, $C_1$- bis $C_4$-Alkansulfonamido-, $C_1$- bis $C_4$-Alkansulfinyl- oder $C_1$- bis $C_4$-Alkansulfonylgruppen,

m die Zahl 2, 3 oder 4,

X und Y gleich oder verschieden sind und jeweils Wasserstoff oder eine Benzylgruppe und

$R_3$ einen zweifach durch $C_1$- bis $C_6$-Alkyl substituierten Phenylrest, einen Nitrophenylrest, einen Aminophenylrest, einen 1,3,5-Tri-$C_1$- bis $C_6$-alkyl-2,4-dioxo-1H, 3H-pyrimidin-6-ylrest, einen Indolylrest, einen Indazolylrest, einen Benzimidazolylrest, einen 1,4-Di-$C_1$- bis $C_6$-alkyl-pyrazol-5-ylrest oder einen Rest Q

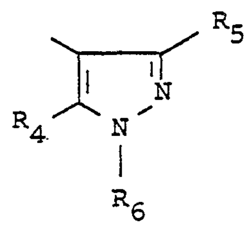

(Q)

worin $R_4$ und $R_5$ gleich oder verschieden sind und jeweils fuer $C_1$- bis $C_4$-Alkylreste stehen und $R_6$ einen Rest aus der folgenden Gruppe : $C_1$- bis $C_6$-Alkyl, $C_1$- bis $C_6$-Alkenyl, Phenyl und Benzyl darstellt, bedeuten, sowie deren pharmakologisch verträgliche Salze, dadurch gekennzeichnet, daß man eine Verbindung der allgemeinen Formel II

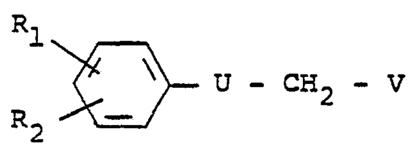

(II)

in der

$R_1$ und $R_2$ die obengenannte Bedeutung haben, waehrend

V einen reaktiven Rest darstellt und

U die Gruppe $>C=O$ oder $>CH-Z$ bedeutet, wobei

Z eine Hydroxygruppe oder auch gemeinsam mit V ein Sauerstoffatom sein kann,

mit einer Verbindung der allgemeinen Formel III

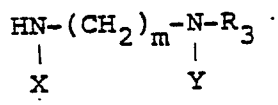

(III)

in der X, Y, m und $R_3$ die obengenannte Bedeutung haben, umsetzt, und für den Fall, daß U die Gruppe $>C=O$ bedeutet, anschließend reduziert, eine für X und/oder Y stehende Benzylgruppe gewünschtenfalls abspaltet, einen für $R_1$ oder $R_2$ stehenden Rest gewünschtenfalls in einen anderen, dem Anspruch entsprechenden Rest umwandelt und gegebenenfalls die so erhaltenen Verbindungen der allgemeinen Formel I in ihre pharmakologisch verträglichen Salze überführt.

6. Arzneimittel, enthaltend eine Verbindung gemäß Ansprüche 1-4 und übliche Träger- und Hilfsstoffe.

7. Verwendung von Verbindungen gemäß Ansprüche 1-4 zur Herstellung von Arzneimitteln zur Behandlung von Herz- und Kreislauferkrankungen.

8. Verwendung von Verbindungen gemäß Ansprüche 1-4 zur Herstellung von Arzneimitteln zur Behandlung von Durchblutungsstörungen.

9. Verbindungen der allgemeinen Formel Ia

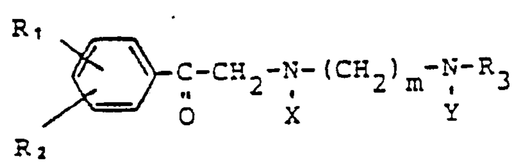

(Ia)

in der

$R_1$ und $R_2$ gleich oder verschieden sind und jeweils Wasserstoff, Hydroxy-, $C_1$- bis $C_4$-Alkoxy-, Benzyloxy-, $C_1$ bis $C_4$-Alkoxycarbonyl-, $C_1$-. bis $C_4$-Alkansulfonamido-, $C_1$- bis $C_4$-Alkansulfinyl- oder $C_1$- bis $C_4$-Alkansulfonylgruppen,

m die Zahl 2, 3 oder 4,

X und Y gleich oder verschieden sind und jeweils Wasserstoff oder eine Benzylgruppe und

$R_3$ einen zweifach durch $C_1$- bis $C_6$-Alkyl substituierten Phenylrest, einen Nitrophenylrest, einen Aminophenylrest, einen 1,3,5-Tri-$C_1$- bis $C_6$-alkyl-2,4-dioxo-1H, 3H-pyrimidin-6-ylrest, einen Indolylrest, einen Indazolylrest, einen Benzimidazolylrest, einen 1,4-Di-$C_1$- bis $C_6$-alkyl-pyrazol-5-ylrest oder einen Rest Q

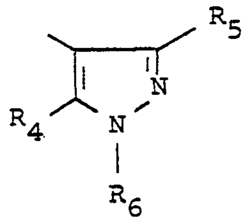

(Q)

worin $R_4$ und $R_5$ gleich oder verschieden sind und jeweils fuer $C_1$- bis $C_4$-Alkylreste stehen und $R_6$ einen Rest aus der folgenden Gruppe : $C_1$- bis $C_6$-Alkyl, $C_1$- bis $C_6$-Alkenyl, Phenyl und Benzyl darstellt, bedeuten.

## Claims

1. Aminoalcohols of the general formula I

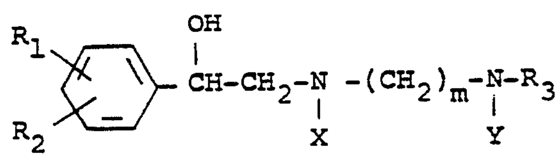

(I)

wherein

$R_1$ and $R_2$ are the same or different and each signifies hydrogen, hydroxyl, $C_1$ to $C_4$-alkoxy, benzyloxy, $C_1$ to $C_4$-alkoxycarbonyl, $C_1$ to $C_4$-alkanesulphonamido, $C_1$ to $C_4$-alkanesulphinyl or $C_1$ to $C_4$-alkanesulphonyl groups,

m the number 2, 3 or 4,

X and Y are the same or different and each signifies hydrogen or a benzyl group and

$R_3$ a phenyl radical substituted twice by $C_1$ to $C_6$-alkyl, a nitrophenyl radical, an aminophenyl radical, a 1,3,5-tri-$C_1$ to $C_6$-alkyl-2,4-dioxo-1H,3H-pyrimidin-6-yl radical, an indolyl radical, an indazolyl radical, a benzimidazolyl radical, a 1,4-di-$C_1$ to $C_6$-alkylpyrazol-5-yl radical or a radical Q

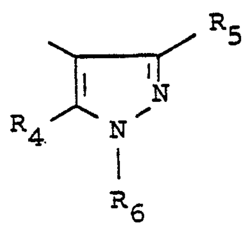

(Q)

wherein $R_4$ and $R_5$ are the same or different and each stand for $C_1$ to $C_4$-alkyl radicals and $R_6$ represents a radical from the following group : $C_1$ to $C_6$-alkyl, $C_1$ to $C_6$-alkenyl, phenyl and benzyl, as well as their pharmacologically acceptable salts.

2. Compounds according to claim 1, in which m, X, Y and $R_3$ have the given meaning and $R_1$ and $R_2$ are the same or different and each represents hydrogen, hydroxyl, methoxy or benzyloxy.

3. Compounds according to claim 1 or 2, in which $R_1$, $R_2$, m, X and Y have the given meaning and $R_3$ represents a 2,6-dimethylphenyl, 3-nitrophenyl, indazolyl, 1,4-dimethylpyrazol-5-yl, 2,4-dioxo-1,3,5-trimethyl-1H,3H-pyrimidin-6-yl radical or a radical Q

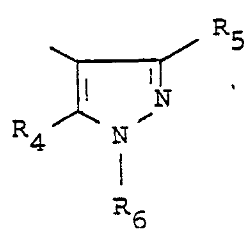

(Q)

wherein $R_4$ and $R_5$ each signify methyl and $R_6$ methyl, allyl, phenyl or benzyl.

4. Compounds according to claim 1, in which

$R_1$ represents hydrogen, hydroxyl, methoxy or benzyloxy,

$R_2$ hydrogen, hydroxyl, methoxy or benzyloxy,

m the numbers 2 or 3,

X hydrogen or benzyl,

Y hydrogen or benzyl and

$R_3$ a 2,6-dimethylphenyl, 3-nitrophenyl, indazolyl, 1,4-dimethylpyrazol-5-yl or 1,3,5-trimethylpyrazol-5-yl radical.

5. Process for the preparation of aminoalcohols of the general formula I

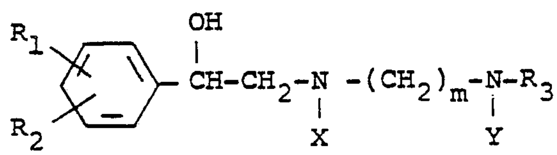

(I)

in which

$R_1$ and $R_2$ are the same or different and each represents hydrogen, hydroxyl, $C_1$ to $C_4$-alkoxy, benzyloxy, $C_1$ to $C_4$-alkoxycarbonyl, $C_1$ to $C_4$-alkanesulphonamido, $C_1$ to $C_4$-alkanesulphinyl or $C_1$ to $C_4$-alkanesulphonyl radicals,

m the number 2, 3 or 4,

X and Y are the same or different and each signifies hydrogen or a benzyl group and

$R_3$ a phenyl radical substituted twice by $C_1$ to $C_6$-alkyl, a nitrophenyl radical, an aminophenyl radical, a 1,3,5-tri-$C_1$ to $C_6$-alkyl-2,4-dioxo-1H,3H-pyrimidin-6-yl radical, an indolyl radical, an indazolyl radical, a benzimidazolyl radical, a 1,4-di-$C_1$ to $C_6$-alkylpyrazol-5-yl radical or a radical Q

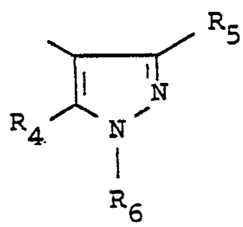

(Q)

wherein $R_4$ and $R_5$ are the same or different and each stands for $C_1$ to $C_4$-alkyl radicals and $R_6$ represents a radical from the following group : $C_1$ to $C_6$-alkyl, $C_1$-$C_6$-alkenyl, phenyl and benzyl ;
as well as of their pharmacologically acceptable salts, characterised in that one reacts a compound of the general formula II

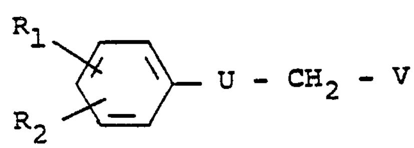

(II)

in which

$R_1$ and $R_2$ have the above-given meaning, whereas

V represents a reactive residue and

U the group ≥C=O or ≥CH—Z, whereby

Z can be a hydroxyl group or also, together with V, can be an oxygen atom,

with a compound of the general formula III

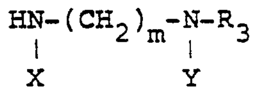

(III)

in which X, Y, m and $R_3$ have the above-given meaning, and, for the case in which U signifies the group ≥C=O, subsequently reduces, or Y, if desired, splits off a benzyl group standing for X and/or Y, if desired, converts a radical standing for $R_1$ or $R_2$ into another radical according to the claim and, if desired, converts the so obtained compounds of general formula I into their pharmacologically acceptable salts.

6. Medicaments containing a compound according to claims 1-4 and conventional carrier and adjuvant materials.

7. Use of compounds according to claims 1-4 for the production of medicaments for the treatment of heart and circulatory diseases.

8. Use of compounds according to claims 1-4 for the production of medicaments for the treatment of blood circulation disturbances.

9. Compounds of the general formula Ia

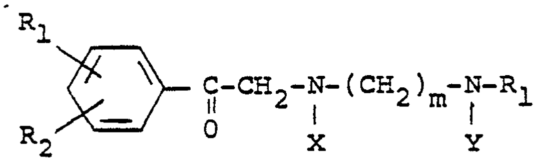

(Ia)

in which

$R_1$ and $R_2$ are the same or different and each signifies hydrogen, hydroxyl, $C_1$ to $C_4$-alkoxy, benzyloxy, $C_1$ to $C_4$-alkoxycarbonyl, $C_1$ to $C_4$-alkanesulphonamido, $C_1$ to $C_4$-alkanesulphinyl or $C_1$ to $C_4$-alkanesulphonyl groups,

m the numbers 2, 3 or 4,

X and Y are the same or different and each signifies hydrogen or a benzyl group and

$R_3$ signifies a phenyl radical substituted twice by $C_1$ to $C_6$-alkyl, a nitrophenyl radical, an aminophenyl radical, a 1,3,5-tri-$C_1$ to $C_6$-alkyl-2,4-dioxo-1H,3H-pyrimidin-6-yl radical, an indolyl radical, an indazolyl radical, a benzimidazolyl radical, a 1,4-di-$C_1$ to $C_6$-alkylpyrazol-5-yl radical or a radical Q

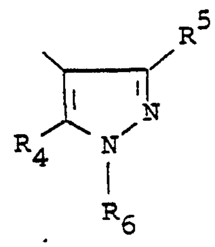

(Q)

wherein $R_4$ and $R_5$ are the same or different and each stands for $C_1$ to $C_4$-alkyl radicals and $R_6$ represents a radical from the following group : $C_1$ to $C_6$-alkyl, $C_1$ to $C_6$-alkenyl, phenyl and benzyl.

**Revendications**

1. Aminoalcools de la formule générale I :

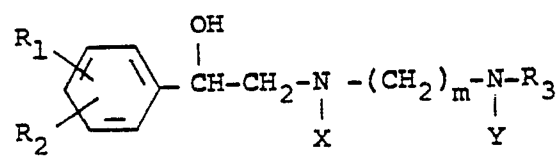

(I)

dans laquelle :

$R_1$ et $R_2$ sont semblables ou différents et représentent chaque fois l'hydrogène, des groupes hydroxyle, un groupe alcoxyle en $C_1$ à $C_4$, benzyloxyle, alcoxycarbonyle en $C_1$ à $C_4$, alcanesulfonamido en $C_1$ à $C_4$, alcanesulfinyle en $C_1$ à $C_4$, ou alcanesulfonyle en $C_1$ à $C_4$,

m le nombre 2, 3 ou 4,

X et Y sont semblables ou différents et représentent chaque fois l'hydrogène ou un groupe benzyle ; et,

$R_3$ un radical phényle deux fois substitué par des groupes alkyle en $C_1$ à $C_6$, un radical nitrophényle, un radical aminophényle, un radical 1,3,5-tris-(alkyle en $C_1$ à $C_6$)-2,4-dioxo-1H,3H-pyrimidine-6-yle, un radical indolyle, un radical indazolyle, un radical benzamidazolyle, un radical 1,4-bis-(alkyle en $C_1$ à $C_6$)-pyrazole-5-yle ou un radical Q :

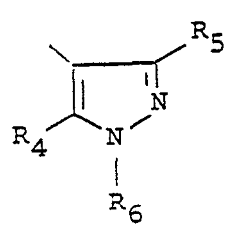

(Q)

dans lequel $R_4$ et $R_5$ sont semblables ou différents et représentent chaque fois des radicaux alkyle en $C_1$ à $C_4$, et $R_6$ un radical du groupe suivant : alkyle en $C_1$ à $C_6$, alcényle en $C_1$ à $C_6$, phényle et benzyle, ainsi que leurs sels pharmacologiquement tolérés.

2. Composés selon la revendication 1, dans lesquels m, X, Y et $R_3$ ont la signification indiquée, et $R_1$ et $R_2$ sont semblables ou différents et représentent chaque fois l'hydrogène, un groupe hydroxyle, méthoxyle ou benzyloxyle.

3. Composés selon l'une des revendications 1 et 2, dans lesquels $R_1$, $R_2$, m, X et Y ont la signification indiquée, et $R_3$ représente un radical 2,6-diméthylphényle, 3-nitrophényle, indazolyle, 1,4-diméthylpyrazole-5-yle, 2,4-dioxo-1,3,5-triméthyl-1H,3H-pyrimidine-6-yl ou un radical Q :

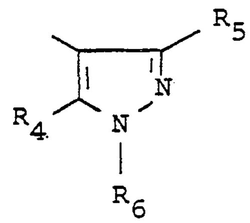

(Q)

dans lequel $R_4$ et $R_5$ représentent chaque fois un groupe méthyle, et $R_6$ un groupe méthyle, allyle, phényle ou benzyle.

4. Composés selon la revendication 1, dans lesquels :

$R_1$ représente l'hydrogène, un groupe hydroxyle, méthoxyle ou benzyloxyle,

$R_2$ l'hydrogène, un groupe hydroxyle, méthoxyle ou benzyloxyle,

m les nombres 2 ou 3,

X l'hydrogène ou un groupe benzyle,

Y l'hydrogène ou un groupe benzyle, et,

$R_3$ un radical 2,6-diméthylphényle, 3-nitrophényle, indazolyle, 1,4-diméthylpyrazole-5-yle ou 1,3,5-triméthylpyrazole-4-yle.

5. Procédé pour la préparation d'aminoalcools de la formule générale I :

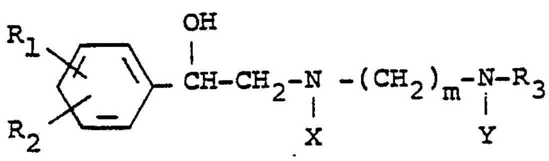

(I)

dans laquelle :

$R_1$ et $R_2$ sont semblables ou différents et représentent chaque fois l'hydrogène, des groupes hydroxyle, un groupe alcoxyle en $C_1$ à $C_4$, benzyloxyle, alcoxycarbonyle en $C_1$ à $C_4$, alcanesulfonamido en $C_1$ à $C_4$, alcanesulfinyle en $C_1$ à $C_4$, ou alcanesulfonyle en $C_1$ à $C_4$,

m le nombre 2, 3 ou 4,

X et Y sont semblables ou différents et représentent chaque fois l'hydrogène ou un groupe benzyle ; et,

$R_3$ un radical phényle deux fois substitué par des groupes alkyle en $C_1$ à $C_6$, un radical nitrophényle, un radical aminophényle, un radical 1,3,5-tris-(alkyle en $C_1$ à $C_6$)-2,4-dioxo-1H,3H-pyrimidine-6-yle, un radical indolyle, un radical indazolyle, un radical benzamidazolyle, un radical 1,4-bis-(alkyle en $C_1$ à $C_6$)-pyrazole-5-yle ou un radical Q :

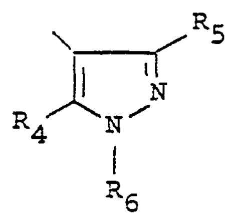

(Q)

dans lequel $R_4$ et $R_5$ sont semblables ou différents et représentent chaque fois des radicaux alkyle en $C_1$ à $C_4$, et $R_6$ un radical du groupe suivant : alkyle en $C_1$ à $C_6$, alcényle en $C_1$ à $C_6$, phényle et benzyle, ainsi que leurs sels pharmacologiquement tolérés, caractérisé par le fait que l'on fait réagir un composé de la formule générale II :

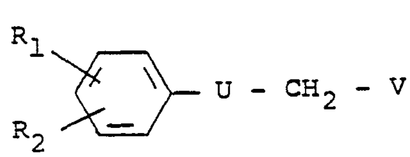

(II)

dans laquelle :

$R_1$ et $R_2$ ont la signification indiquée plus haut, tandis que

V représente un radical réactif, et,

U le groupe >C=O ou >CH—Z,

Z pouvant être un groupe hydroxyle ou encore, conjointement avec V, un atome d'oxygène, sur un composé de la formule générale III :

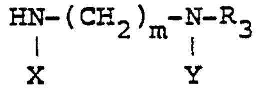

(III)

dans laquelle X, Y, m, et $R_3$ ont la signification indiquée plus haut, et que, pour le cas où U représente le groupe >C=O, on le réduit ensuite, que l'on élimine, si on le désire, un groupe benzyle représentant X et/ou Y, que l'on convertit, si on le désire, un radical représentant $R_1$ ou $R_2$ en un autre radical conforme à la revendication, et qu'éventuellement, on convertit les composés de la formule générale I ainsi obtenus en leurs sels pharmacologiquement tolérés.

6. Médicament contenant un composé selon les revendications 1 à 4 et des véhicules et adjuvants usuels.

7. Utilisation de composés selon les revendications 1 à 4 pour la préparation de médicaments pour le traitement de maladies du cœur et de la circulation.

8. Utilisation de composés selon les revendications 1 à 4 pour la fabrication de médicaments pour le traitement de troubles de l'irrigation sanguine.

9. Composés de la formule générale Ia :

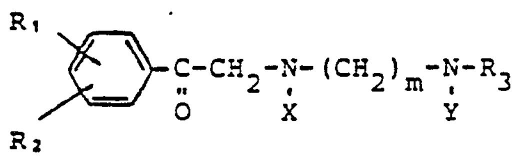

(Ia)

dans laquelle :

$R_1$ et $R_2$ sont semblables ou différents et représentent chaque fois l'hydrogène, des groupes hydroxyle, alcoxyle en $C_1$ à $C_4$, benzyloxyle, alcoxycarbonyle en $C_1$ à $C_4$, alcanesulfonamido en $C_1$ à $C_4$, alcanesulfinyle en $C_1$ à $C_4$, ou alcanesulfonyle en $C_1$ à $C_4$,

m le nombre 2, 3 ou 4,

X et Y sont semblables ou différents et représentent chaque fois l'hydrogène ou un groupe benzyle, et,

$R_3$ un radical phényle deux fois substitué par des groupes alkyle en $C_1$ à $C_6$, un radical nitrophényle, un radical aminophényle, un radical 1,3,5-tris-(alkyle en $C_1$ à $C_6$)-2,4-dioxo-1H,3H-pyrimidine-6-yle, un radical indolyle, un radical indazolyle, un radical benzimidazolyle, un radical 1,4-bis-(alkyle en $C_1$ à $C_6$)-pyrazole-5-yle), ou un radical Q :

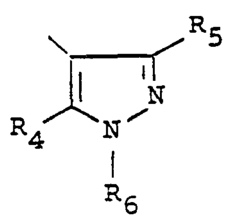

(Q)

dans lequel $R_4$ et $R_5$ sont semblables ou différents et représentent chaque fois des radicaux alkyle en $C_1$ à $C_4$, et $R_6$ un radical du groupe suivant : alkyle en $C_1$ à $C_6$, alcényle en $C_1$ à $C_6$, phényle et benzyle.